# EUROPEAN PATENT SPECIFICATION

(11) **EP 3 384 039 B1**
(45) Date of publication and mention of the grant of the patent: **30.04.2025**
(21) Application number: 16805902.0
(22) Date of filing: 29.11.2016
(51) Int. Cl.: C12P 19/56, C12Q 1/48, C12N 15/52, C12N 9/10

(54) **PROCESS FOR PRODUCING HIGH PURITY STEVIOL GLYCOSIDES**
VERFAHREN ZUR HERSTELLUNG VON HOCHREINEN STEVIOL-GLYCOSIDEN
PROCÉDÉ DE PRODUCTION DE GLYCOSIDES DE STÉVIOL D'UNE GRANDE PURETÉ

(30) Priority: 30.11.2015 US 201562261242 P
(43) Date of publication of application: 10.10.2018
(73) Proprietor: PureCircle SDN BHD, 71760 Bandar Enstek, Negeri Sembilan (MY)
(72) Inventor: MARKOSYAN, Avetik, 0014 Yerevan (AM); BUESCHER, Joerg, 79194 Gundelfingen (DE); MEURER, Guido, 64342 Seeheim-Jugenheim (DE); ZUREK, Petra, 64653 Lorsch (DE)
(74) Representative: Novagraaf Group
(86) International application number: PCT/IB2016/057188
(87) International publication number: WO 2017/093895

(56) References cited:
- EP-A1- 3 034 614
- EP-A1- 3 101 139
- WO-A1-2013/176738
- WO-A1-2016/168413
- WO-A2-2013/022989
- WO-A2-2014/122227
- WO-A2-2015/065650
- CN-A- 103 031 283
- INDRA PRAKASH ET AL: "Isolation and Characterization of a Novel Rebaudioside M Isomer from a Bioconversion Reaction of Rebaudioside A and NMR Comparison Studies of Rebaudioside M Isolated from Stevia rebaudiana Bertoni and Stevia rebaudiana Morita", BIOMOLECULES, vol. 4, no. 2, 31 March 2014 (2014-03-31), pages 374 - 389, XP055237016, DOI: 10.3390/biom4020374

## Description

### BACKGROUND OF THE INVENTION

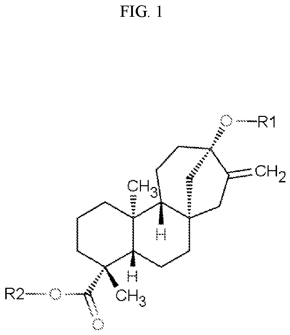

Steviol glycosides are a class of compounds that occur naturally in plants of the species *Stevia rebaudiana* and that are of commercial interest due to their intense sweet taste. Extracts of *S. rebaudiana* are marketed as zero calorie sweeteners for use in food and beverages. These extracts contain a mixture of steviol glycosides that differ in their taste properties. All share a common aglycon (FIG. 1) and differ in the number and position of glucose residues.

Rebaudioside A (RebA) is one of the main components of commercially available stevia extracts. In RebA, the aglycon carries three glucose residues on the R1 position and one glucose residue on the R2 position. Rebaudioside D (RebD) and Rebaudioside M (RebM) have superior taste properties compared to RebA, but only occur in small amounts in stevia extracts. RebD carries two glucose residues on R2 and RebM carries three glucose residues on R2, respectively. They can be presumably synthesized from RebA in reactions that are catalyzed by UDP-glycosyl transferases (UGTs). These enzymes require UDP-glucose as a co-substrate. This compound is highly instable and costly, therefore it needs to be regenerated. Many living cells possess the ability to regenerate UDP-glucose intracellularly.

To efficiently produce RebD and/or RebM a host cell is required that can take up RebA and regenerate UDP glucose and that can be engineered to produce UGT enzymes that catalyze the required glycosylation reactions. Preferably, the species that the host cell belongs to a microbial species that has a history of safe use in food or beverage products and has the ability to grow as single cells in simple culture media.

Previously, it was unclear if any microbe has the ability to assimilate RebA. RebA is a very rare compound in nature; to date only two plant species have been discovered that produce this compound (Philippe et al., 2014). Studies on the degradation of RebA by the gut microbiome indicate that the glucose residues are cleaved off and that the remaining steviol is not metabolized further (Gardana et al., 2003). This indicates that the glucose residues are cleaved off by extracellular (secreted) enzymes and subsequently the free glucose is assimilated.

### BRIEF DESCRIPTION OF THE DRAWINGS

FIG. 1 shows the structure of an aglycon of steviol glycosides.
FIG. 2 is a bar graph showing the concentration of Rebaudioside A in quench, wash, and cell extract solutions of a *Kluyveromyces marxianus* strain.

### DETAILED DESCRIPTION

The present invention is defined by the claims. Accordingly the present invention relates to a method of producing steviol glycosides, comprising the step of intracellularly converting rebaudioside A to rebaudioside D, rebaudioside M, or a combination thereof, wherein the step of intracellularly converting rebaudioside A includes subjecting rebaudioside A to a UDP-glucosyl transferase enzyme, the UDP-glucosyl transferase enzyme is intracellularly produced by a host cell, the host cell is capable of rebaudioside A uptake from a culture medium and is capable of expressing genes of enzymes for intracellular conversion of rebaudioside A to rebaudioside D, rebaudioside M, or a combination thereof and wherein the host cell is of a species selected from: *Candida utilis, Cyberlindnera jadinii, Kluyveromyces lactis, Kluyveromyces marxianus, Meyerozyma guilliermondii, Pichia guilliermondii, Pichia jadinii, and Zygosaccharomyces rouxii.*

### Initial Screening

Candidate microbial strains were chosen based on strains belonging to species that have been described to be present in non-spoiled food or that have been previously used in biotechnological processes, and were ranked with respect to 1.eukaryote or prokaryote, 2. availability of molecular biology protocols, and 3. biological safety level. Suitable genera for selecting candidate microbial strains include *Candida, Cyberlindnera, Kluyveromyces, Meyerozyma, Pischia., Rhodosporidium, Zygosaccharomyces, Saccharomyces, Aspergillus, Hansenula, Humicola, Trichosporon, Brettanomyces, Pachysolen, Yarrowia, Yamadazyma, Schizosaccharomyces, Ashbya, Cyberlindnera, Pichia, Arxula, Xanthophyllomyces or Escherichia.* In certain implementations, *Arthrobacter globiformis, Aspergillus niger, Aspergillus oryzae, Bacillus licheniformis, Bacillus sphaericus, Bacillus subtilis, Brevibacterium linens, Candida utilis, Candida vini, Corynebacterium glutamicum, Cyberlindnera jadinii, Cyberlindnera* sp., *Debaryomyces hansenii, Fusarium semitectum, Hypomyces armeniacus, Kluyveromyces lactis, Kluyveromyces marxianus, Kocuria rhizophila, Lactobacillus brevis, Lactobacillus casei, Lactobacillus pentoses, Lactobacillus plantarum, Lactobacillus reuteri, Meyerozyma guilliermondii, Microbacterium* sp., *Micrococcus luteus, Mucor hiemalis, Mucor racemosus, Penicillium roqueforti, Pichia guilliermondii, Pichia jadinii, Pichia pastoris, Pseudomonas fluorescens, Pseudomonas stutzeri, Rhodosporidium* sp., *Rhodosporidium toruloides, Rhodotorula mucilaginosa, Rhodotorula rubra, Rhodotorula* sp., *Saccharomyces bayanus, Saccharomyces cerevisiae, Saccharomyces pastorianus, Streptomyces albus, Streptomyces coelicolor, Streptomyces griseus, Streptomyces lividans, Torulaspora delbrueckii, Trichosporon laibachii, Trichosporon oleaginosus, Yarrowia lipolytica, Zygosaccharomyces rouxii, Zymomonas mobilis* strains are useful.

Strains were cultivated on diluted complex medium containing (per liter) 1 g yeast extract, 2 g peptone, 10 g RebA. A volume of 1 mL culture medium was inoculated with a single colony.

All cultivations were performed in 96 square well plates on a laboratory shaker with 300 rpm and 5.1 cm shaking diameter at 28 °C for 72 h.

After cultivation, cells were separated from the culture supernatant by centrifugation at 5311 g for 5 min. The supernatant was filtered through a 0.45 µm hPTFE filter and subsequently incubated for 48 h at 28 °C. Then the pelleted cells were re-suspended in Buffer U containing (per liter) 300 mM potassium, 50 mM phosphate, 245 mM glutamate, 20 mM sodium, 2 mM manganese, 0.5 mM calcium, adjusted to pH 6.8 with potassium hydroxide. Subsequently this suspension was centrifuged again at 5311 g for 5 min. The supernatant was discarded and the cell pellets were resuspended in buffer U. Then cells were disrupted for 2 min in a Mini Bead Beater (Biospec Produkts). Cell debris was separated from the soluble fraction by centrifugation for 4 min 4870 g. The supernatant was filtered through a 0.45 µm HPTFE filter. Then, 70 µL of a 2 g/L RebA solution was added to 70 µl of the soluble fraction before incubation at 48 h at 28 °C. Samples were taken at the beginning and at the end of the incubation and RebB was quantified by LC-MS.

20 µL of samples for LC-MS was added to 980 µL of a stopping solution containing 70% acetonitrile, 29.9% water and 0.1% formic acid. Stopped samples were filtered through a 0.45 µm hPTFE filter. Five µL of the filtered samples were used for each injection. Separation was achieved using TSKgel Amide-80 column with dimensions 4.6 x 250mm and a isocratic flow of 70% acetonitrile, 19.9% water, 0.01% formic acid, and 10% 100 mM amonium acetate in water. RebB was detected by LCMS 2010EV mass spectrometry (Shimadzu) set to single ion monitoring (SIM) mode, negative mode, m/z = 803.50.

The difference in RebB concentration between the start and the end of the incubation was calculated for each supernatant and each soluble fraction. This difference value was compared to sterile controls that were processed the same way. A large difference in the soluble fraction and a small difference in supernatant indicate that a strain might have taken up RebA and degraded it to RebB to use the thus liberated glucose molecule to support growth.

### Indirect Assay

Microbial strains were assayed for their ability to assimilate RebA from the culture medium by growing the strains in a defined mineral medium with RebA as the only carbon source. 19 microbial strains from *Candida utilis, Cyberlindnera jadinii, Kluyveromyces lactis, Kluyveromyces marxianus, Meyerozyma guilliermondii, Pichia guilliermondii, Pichia jadinii,* and *Zygosaccharomyces rouxii* were found to assimilate RebA .

This medium contained (per liter): 10 g RebA, 5 g (NH₄)₂SO₄, 3 g KH₂PO₄, 0.5 g MgSO₄ × 7H₂O,15 mg EDTA, 4.5 mg ZnSO₄ × 7H₂O, 0.3 mg CoCl₂ × 6H₂O, 1 mg MnCl₂ × 4H₂O, 0.3 mg CuSO₄ ×5H₂O, 4.5 mg CaCl₂ × 2H₂O, 3 mg FeSO₄ × 7H₂O, 0.4 mg NaMoO₄ × 2H₂O, 1 mg H₃BO₃, 0.1 mg KI, 0.05 mg biotin, 1 mg calcium pantothenate, 25 mg inositol, 1 mg thiamine HCl, 1 mg pyridoxine HCl, 0.2 mg para-aminobenzoic acid. A volume of 1 mL culture medium was inoculated with 10 µl of an overnight culture grown.

All cultivations were performed in 96 square well plates on a laboratory shaker with 300 rpm and 5.1 cm shaking diameter at 28 °C for 72 h. Growth was monitored by mixing 50 µL or culture volume with 50 µL ethanol in a 96 well plate and subsequently measuring the optical density at 600 nm (OD600) in a spectrophotometer (Molecular Devices). The OD600 values that were observed in wells that were inoculated were compared to the OD600 values that were observed in wells that contained culture medium only. If the OD600 exceeded that in the sterile reference wells the strain was identified to grow with RebA as the only carbon source.

After cultivation, cells were separated from the culture supernatant by centrifugation at 4025 g for 5 min. The cell pellets were re-suspended in buffer U. Then cells were disrupted for 2 min using a Mini Bead Beater (Biospec Produkts). Cell debris was separated from the soluble fraction by centrifugation for 4 min at 4870 g. The supernatant was filtered through a 0.45 µm HPTFE filter.

Then, 70 µL of a 2 g/L RebA solution was added to the soluble fraction before incubation at 72 h at 28 °C. RebA was detected by LC-MS set to single ion monitoring (SIM) mode, negative mode, m/z = 965.60.

The difference in RebA concentration between the start and the end of the incubation was calculated for each soluble fraction. If RebA can be degraded by the soluble fraction of the cell extract, it can be assumed that the strain can assimilate RebA from the culture medium.

### Direct Assay

For one of the *Kluyveromyces marxianus* strains found to assimilate RebA by the indirect assay, RebA assimilation was demonstrated by quantification of intracellular RebA.

The selected microbial strain was cultivated in a defined mineral medium with RebA as the only carbon source. This medium contained (per liter): 9 g RebA, 5 g (NH₄)₂SO₄, 3 g KH₂PO₄, 0.5 g MgSO₄ x 7H₂O, 15 mg EDTA, 4.5 mg ZnSO₄ x 7H₂O, 0.3 mg CoCl₂ x 6H₂O, 1 mg MnCl₂ x 4H₂O, 0.3 mg CuSO₄ x 5H₂O, 4.5 mg CaCl₂ x 2H₂O, 3 mg FeSO₄ x 7H₂O, 0.4 mg NaMoO₄ x 2H₂O, 1 mg H₃BO₃, 0.1 mg KI, 0.05 mg biotin, 1 mg calcium pantothenate, 25 mg inositol, 1 mg thiamine HCl, 1 mg pyridoxine HCl, 0.2 mg para-aminobenzoic acid. A volume of 20 mL culture medium was inoculated with a single colony.

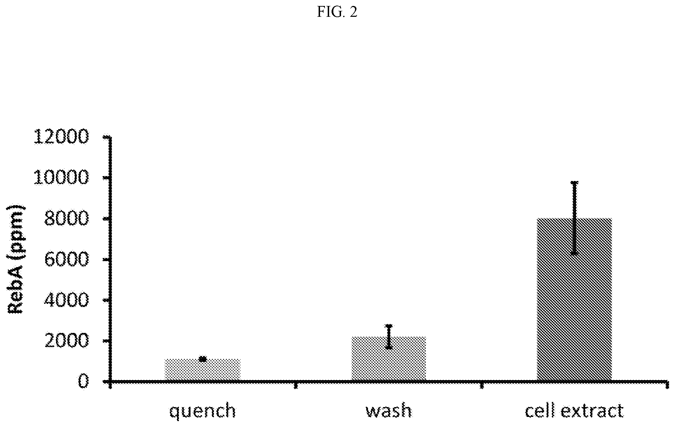

All cultivations were performed in 25 mL culture medium in a 300 mL non-baffled shake flask on a laboratory shaker with 250 rpm and 2.5 cm shaking diameter at 28 °C for 72 h. The complete culture was transferred to a beaker containing 125 mL of a solution containing 45 ppm 4- Nitrophenyl β-D-glucopyranoside in 60% methanol in water that was pre-cooled to -40 °C. The mixture was then transferred to three 50 mL centrifugation tubes that were pre-cooled on dry ice and centrifuged below 0 °C at 4248 g for 10 min. Subsequently, an aliquot of the supernatant (quench) was stored at -80 °C and the rest of the supernatant was discarded, while the pellet was kept on dry ice. Then 5 mL 60% methanol in water pre-cooled on dry ice was used to re-suspend the 3 pellets. The re-suspended pellets were transferred to a single 15 mL centrifugation tube precooled on dry ice and centrifuged below 0 °C at 4248 g for 10 min. Then the supernatant was discarded and the pellet was re-suspended in fresh 5 mL 60% methanol in water pre-cooled on dry ice and the suspension was centrifuged below 0 °C at 4248 g for 10 min. Then the supernatant was discarded and the pellet was re-suspended again in fresh 5 mL 60% methanol in water pre-cooled on dry ice and the suspension was centrifuged below 0 °C at 4248 g for 10 min. Then the supernatant (wash) was separated and stored at -80 °C. Then the pellet was re-suspended in 1 mL 60% ethanol in water pre-heated to 78 °C and incubated at 78 °C for 3 minutes. Then the suspension was cooled on dry ice and transferred to a 2 mL centrifugation tube and centrifuged for 3 min at 16500 g at room temperature. The supernatant (cell extract) was separated and stored at -80 °C. The concentration of RebA in quench, wash, and cell extract was quantified by LC-MS (FIG. 2), in which the error bars indicate the standard error of mean from 2 parallel experiments. The concentration of RebA in the cell extract exceeds that in the quench and wash solutions.

Consequently, RebA has been accumulated intracellularly. The RebA concentration in the wash solution is higher than in the quench solution, which indicates that some RebA leaked from the pellet during sample processing.

In one implementation of the disclosure a microbial strain capable of assimilating RebA hosts expressed genes and respective enzymes for intracellular conversion of RebA to RebD and/or Reb M.

In another implementation the said enzymes include at least one UDP-glucosyltransferase (UGT).

In yet another implementation the said enzymes include at least one sucrose synthase for UDP regeneration and recycling.

In another implementation the microbial strain is capable of excreting the intracellular Reb *D* and/or Reb *M*.

In yet another implementation the Reb *D* and/or Reb *M* synthesized by microbial strain of this disclosure is recovered and purified by techniques used in steviol glycosides' extraction and purification to provide steviol glycosides compositions comprising the Reb *D* and/or Reb *M.*

In another implementation steviol glycosides compositions of present disclosure can be used as sweeteners, sweetness enhancers, flavors and flavor enhancers in various food and beverage products. Non-limiting examples of food and beverage products include carbonated soft drinks, including but not limited to cola flavored carbonated soft drinks, fruit flavored carbonated soft drinks, berry flavored carbonated soft drinks, ready to drink beverages, energy drinks, isotonic drinks, low-calorie drinks, zero-calorie drinks, sports drinks, teas, fruit and vegetable juices, juice drinks, dairy drinks, yoghurt drinks, alcohol beverages, powdered beverages, bakery products, cookies, biscuits, baking mixes, cereals, confectioneries, candies, toffees, chewing gum, dairy products, flavored milk, yoghurts, flavored yoghurts, cultured milk, soy sauce and other soy base products, salad dressings, mayonnaise, vinegar, frozen-desserts, meat products, fish-meat products, bottled and canned foods, tabletop sweeteners, fruits and vegetables.

Additionally the steviol glycosides compositions of present disclosure can be used in drug or pharmaceutical preparations and cosmetics, including but not limited to toothpaste, mouthwash, cough syrup, chewable tablets, lozenges, vitamin preparations, and the like.

The steviol glycosides compositions of present disclosure can be used "as-is" or in combination with other sweeteners, flavors and food ingredients.

Non-limiting examples of sweeteners include rebaudioside *A,* rebaudioside *B,* rebaudioside *C*, rebaudioside *D,* rebaudioside E, rebaudioside *F*, rebaudioside *G*, rebaudioside *H,* rebaudioside *I,* rebaudioside *J*, rebaudioside *K,* rebaudioside *L,* rebaudioside *M,* rebaudioside *N,* rebaudioside *O*, dulcoside *A*, steviolbioside, rubusoside, as well as other steviol glycosides found in *Stevia rebaudiana* plant and mixtures thereof, stevia extracts, glycosylated steviol glycosides, steviol glycosides prepared by chemical, enzymatic synthesis or by fermentation of recombinant microorganisms, Luo Han Guo extract, mogrosides, glycosylated mogrosides, high-fructose corn syrup, corn syrup, invert sugar, fructooligosaccharides, inulin, inulooligosaccharides, coupling sugar, maltooligosaccharides, maltodextrins, dextrins, limited dextrins, corn syrup solids, glucose, maltose, sucrose, lactose, allulose, tagatose, aspartame, saccharin, sucralose, sugar alcohols and mixtures thereof.

Non-limiting examples of flavors include cola, lemon, lime, orange, grapefruit, banana, grape, apple, pear, pineapple, bitter almond, cinnamon, sugar, cotton candy, vanilla flavors, glycosylated steviol glycosides, NSF02 and mixtures thereof.

Non-limiting examples of other food ingredients include flavors, acidulants, organic and amino acids, coloring agents, bulking agents, modified starches, gums, texturizers, preservatives, antioxidants, vitamins, emulsifiers, stabilisers, thickeners, gelling agents caffeine, theanine, theobromine and mixtures thereof.

## Claims

1. A method of producing steviol glycosides, comprising the step of intracellularly converting rebaudioside A to rebaudioside D, rebaudioside M, or a combination thereof, wherein the step of intracellularly converting rebaudioside A includes subjecting rebaudioside A to a UDP-glucosyl transferase enzyme, the UDP-glucosyl transferase enzyme is intracellularly produced by a host cell, the host cell is capable of rebaudioside A uptake from a culture medium and is capable of expressing genes of enzymes for intracellular conversion of rebaudioside A to rebaudioside D, rebaudioside M, or a combination thereof and wherein the host cell is of a species selected from: *Candida utilis, Cyberlindnera jadinii, Kluyveromyces lactis, Kluyveromyces marxianus, Meyerozyma guilliermondii, Pichia guilliermondii, Pichia jadinii, and Zygosaccharomyces rouxii.*

2. The method of claim 1, wherein the host cell is capable of producing sucrose synthase for UDP regeneration and recycling.

3. The method of claim 1, wherein the host cell is identified by a process comprising the steps of:
(a) providing a culture medium comprising rebaudioside A as carbon source;
(b) cultivating a candidate microbial strain in the culture medium;
(c) separating cells of the candidate microbial strain from the culture medium to result in a supernatant, analyzing the supernatant for rebaudioside B, and then incubating the supernatant;
(d) disrupting the cells to provide a soluble fraction and a cell debris fraction;
(e) adding rebaudioside A to the soluble fraction, analyzing the soluble fraction for rebaudioside B, and then incubating the soluble fraction;
(f) analyzing the incubated supernatant and the incubated soluble fraction for rebaudioside B;
(g) identifying the host cell by determining the presence of a greater amount of rebaudioside B in the incubated soluble fraction than in the incubated supernatant.

4. The method of claim 1, wherein the host cell is identified by a process comprising the steps of:
(a) providing a culture medium comprising rebaudioside A as only carbon source;
(b) cultivating a candidate microbial strain in the culture medium;
(c) separating cells of the microbial strain from the culture medium;
(d) re-suspending the cells and disrupting them to form a soluble fraction and a cell debris fraction;
(e) adding rebaudioside A to the soluble fraction, analyzing the soluble fraction for rebaudioside A, and then incubating the soluble fraction;
(f) analyzing the incubated soluble fraction for rebaudioside A;
(g) identifying the host cell by determining a decrease in the amount of rebaudioside A in the incubated soluble fraction as compared to the soluble fraction.

5. The method of claim 1, wherein the host cell is identified by a process comprising the steps of:
(a) providing a culture medium comprising rebaudioside A as a carbon source;
(b) cultivating a candidate microbial strain in the culture medium;
(c) adding a methanol solution to the culture medium, resulting in a mixture;
(d) centrifuging the mixture to separate cells of the microbial strain, resulting in a first pellet and a quench supernatant;
(e) re-suspending the first pellet in methanol to form a first suspension and centrifuging the first suspension to result in a second pellet and a wash supernatant;
(f) re-suspending the second pellet in ethanol to form a second suspension, and incubating the second suspension;
(g) centrifuging the second suspension to form a supernatant cell extract;
(h) analyzing each of the cell extract supernatant, the wash supernatant, and the quench supernatant for a rebaudioside A content; and
(i) identifying the host cell by determining a greater concentration of rebaudioside A in the cell extract supernatant than in the wash supernatant and the quench supernatant.

6. The method of claim 5, wherein the host cell is of the species *Kluyveromyces marxianus.*

## Patentansprüche

1. Verfahren zum Herstellen von Steviolglycosiden, umfassend den Schritt der intrazellulären Umwandlung von Rebaudiosid A in Rebaudiosid D, Rebaudiosid M oder eine Kombination davon, wobei der Schritt der intrazellulären Umwandlung von Rebaudiosid A die Behandlung von Rebaudiosid A mit einem UDP-Glucosyltransferase-Enzym einschließt, wobei das UDP-Glucosyltransferase-Enzym intrazellulär von einer Wirtszelle produziert wird, wobei die Wirtszelle in der Lage ist, Rebaudiosid A aus einem Kulturmedium aufzunehmen und Gene von Enzymen zur intrazellulären Umwandlung von Rebaudiosid A in Rebaudiosid D, Rebaudiosid M oder eine Kombination davon zu exprimieren, und wobei die Wirtszelle einer Spezies angehört, ausgewählt aus: *Candida utilis, Cyberlindnera jadinii, Kluyveromyces lactis, Kluyveromyces marxianus, Meyerozyma guilliermondii, Pichia guilliermondii, Pichia jadinii und Zygosaccharomyces rouxii.*

2. Verfahren nach Anspruch 1, wobei die Wirtszelle in der Lage ist, Saccharosesynthase zur UDP-Regeneration und zum UDP-Recycling zu produzieren.

3. Verfahren nach Anspruch 1, wobei die Wirtszelle durch einen Prozess identifiziert wird, umfassend die Schritte:
(a) Bereitstellen eines Kulturmediums, umfassend Rebaudiosid A als Kohlenstoffquelle;
(b) Kultivieren eines mikrobiellen Kandidatenstammes in dem Kulturmedium;
(c) Trennen der Zellen des mikrobiellen Kandidatenstammes von dem Kulturmedium, um einen Überstand zu ergeben, Analysieren des Überstands auf Rebaudiosid B und anschließendes Inkubieren des Überstands;
(d) Aufbrechen der Zellen, um eine lösliche Fraktion und eine Zelltrümmerfraktion bereitzustellen;
(e) Hinzufügen von Rebaudiosid A zu der löslichen Fraktion, Analysieren der löslichen Fraktion auf Rebaudiosid B und anschließendes Inkubieren der löslichen Fraktion;
(f) Analysieren des inkubierten Überstands und der inkubierten löslichen Fraktion auf Rebaudiosid B;
(g) Identifizieren der Wirtszelle durch Feststellen des Vorhandenseins einer größeren Menge an Rebaudiosid B in der inkubierten löslichen Fraktion als in dem inkubierten Überstand.

4. Verfahren nach Anspruch 1, wobei die Wirtszelle durch einen Prozess identifiziert wird, umfassend die Schritte:
(a) Bereitstellen eines Kulturmediums, umfassend Rebaudiosid A als einzige Kohlenstoffquelle;
(b) Kultivieren eines mikrobiellen Kandidatenstammes in dem Kulturmedium;
(c) Trennen der Zellen des mikrobiellen Stammes von dem Kulturmedium;
(d) Resuspendieren der Zellen und Aufbrechen derselben, um eine lösliche Fraktion und eine Zelltrümmerfraktion zu bilden;
(e) Hinzufügen von Rebaudiosid A zu der löslichen Fraktion, Analysieren der löslichen Fraktion auf Rebaudiosid A und anschließendes Inkubieren der löslichen Fraktion;
(f) Analysieren der inkubierten löslichen Fraktion auf Rebaudiosid A;
(g) Identifizieren der Wirtszelle durch Feststellen der Abnahme der Menge an Rebaudiosid A in der inkubierten löslichen Fraktion verglichen mit der löslichen Fraktion.

5. Verfahren nach Anspruch 1, wobei die Wirtszelle durch einen Prozess identifiziert wird, umfassend die Schritte:
(a) Bereitstellen eines Kulturmediums, umfassend Rebaudiosid A als eine Kohlenstoffquelle;
(b) Kultivieren eines mikrobiellen Kandidatenstammes in dem Kulturmedium;
(c) Hinzufügen einer Methanollösung zu dem Kulturmedium, wodurch eine Mischung entsteht;
(d) Zentrifugieren der Mischung, um die Zellen des mikrobiellen Stammes abzutrennen, wodurch ein erstes Pellet und ein gequenchter Überstand entstehen;
(e) Resuspendieren des ersten Pellets in Methanol, um eine erste Suspension zu bilden, und Zentrifugieren der ersten Suspension, um ein zweites Pellet und einen Waschüberstand zu ergeben;
(f) Resuspendieren des zweiten Pellets in Ethanol, um eine zweite Suspension zu bilden, und Inkubieren der zweiten Suspension;
(g) Zentrifugieren der zweiten Suspension, um einen überstehenden Zellextrakt zu bilden;
(h) Analysieren des Zellextraktüberstands, des Waschüberstands und des gequenchten Überstands auf einen Rebaudiosid-A-Gehalt; und
(i) Identifizieren der Wirtszelle durch Feststellen einer höheren Konzentration von Rebaudiosid A im Zellextraktüberstand als im Waschüberstand und im gequenchten Überstand.

6. Verfahren nach Anspruch 5, wobei die Wirtszelle von der Spezies *Kluyveromyces marxianus* ist.

## Revendications

1. Procédé de production de glycosides de stéviol, comprenant l'étape de conversion intracellulaire du rébaudioside A en rébaudioside D, rébaudioside M, ou une combinaison de ceux-ci, dans lequel l'étape de conversion intracellulaire du rébaudioside A comporte le fait de soumettre du rébaudioside A à une enzyme UDP-glucosyltransférase, l'enzyme UDP-glucosyltransférase est produite de manière intracellulaire par une cellule hôte, la cellule hôte est capable d'absorber le rébaudioside A à partir d'un milieu de culture et est capable d'exprimer des gènes d'enzymes pour la conversion intracellulaire du rébaudioside A en rébaudioside D, rébaudioside M, ou une combinaison de ceux-ci, et dans lequel la cellule hôte provient d'une espèce choisie parmi *: Candida utilis, Cyberlindnera jadinii, Kluyveromyces lactis, Kluyveromyces marxianus, Meyerozyma guilliermondii, Pichia guilliermondii, Pichia jadinii, et Zygosaccharomyces rouxii.*

2. Procédé selon la revendication 1, dans lequel la cellule hôte est capable de produire de la sucrose synthase pour la régénération et le recyclage d'UDP.

3. Procédé selon la revendication 1, dans lequel la cellule hôte est identifiée par un processus comprenant les étapes consistant à :
(a) fournir un milieu de culture comprenant du rébaudioside A comme source de carbone ;
(b) cultiver une souche microbienne candidate dans le milieu de culture ;
(c) séparer des cellules de la souche microbienne candidate du milieu de culture pour obtenir un surnageant, analyser le surnageant pour la présence de rébaudioside B, puis incuber le surnageant ;
(d) perturber les cellules pour fournir une fraction soluble et une fraction de débris cellulaires ;
(e) ajouter du rébaudioside A à la fraction soluble, analyser la fraction soluble pour la présence de rébaudioside B, puis incuber la fraction soluble ;
(f) analyser le surnageant incubé et la fraction soluble incubée pour la présence de rébaudioside B ;
(g) identifier la cellule hôte en déterminant la présence d'une plus grande quantité de rébaudioside B dans la fraction soluble incubée que dans le surnageant incubé.

4. Procédé selon la revendication 1, dans lequel la cellule hôte est identifiée par un processus comprenant les étapes consistant à :
(a) fournir un milieu de culture comprenant du rébaudioside A comme seule source de carbone ;
(b) cultiver une souche microbienne candidate dans le milieu de culture ;
(c) séparer des cellules de la souche microbienne du milieu de culture ;
(d) remettre les cellules en suspension et les perturber pour former une fraction soluble et une fraction de débris cellulaires ;
(e) ajouter du rébaudioside A à la fraction soluble, analyser la fraction soluble pour la présence de rébaudioside A, puis incuber la fraction soluble ;
(f) analyser la fraction soluble incubée pour la présence de rébaudioside A ;
(g) identifier la cellule hôte en déterminant une diminution de la quantité de rébaudioside A dans la fraction soluble incubée par rapport à la fraction soluble.

5. Procédé selon la revendication 1, dans lequel la cellule hôte est identifiée par un processus comprenant les étapes consistant à :
(a) fournir un milieu de culture comprenant du rébaudioside A comme source de carbone ;
(b) cultiver une souche microbienne candidate dans le milieu de culture ;
(c) ajouter une solution de méthanol au milieu de culture, conduisant à un mélange ;
(d) centrifuger le mélange pour séparer des cellules de la souche microbienne, conduisant à un premier culot et à un surnageant de désactivation ;
(e) remettre le premier culot en suspension dans du méthanol pour former une première suspension et centrifuger la première suspension pour obtenir un second culot et un surnageant de lavage ;
(f) remettre le second culot en suspension dans de l'éthanol pour former une seconde suspension, et incuber la seconde suspension ;
(g) centrifuger la seconde suspension pour former un extrait cellulaire surnageant ;
(h) analyser la teneur en rébaudioside A de chacun du surnageant d'extrait cellulaire, du surnageant de lavage, et du surnageant de désactivation ; et
(i) identifier la cellule hôte en déterminant une plus grande concentration en rébaudioside A dans le surnageant d'extrait cellulaire que dans le surnageant de lavage et le surnageant de désactivation.

6. Procédé selon la revendication 5, dans lequel la cellule hôte provient de l'espèce *Kluyveromyces marxianus.*
